# EUROPÄISCHE PATENTANMELDUNG

(11) **EP 4 732 810 A1**
(43) Veröffentlichungstag der Anmeldung: **29.04.2026**
(21) Anmeldenummer: 24208247.7
(22) Anmeldetag: 23.10.2024
(51) Int. Cl.: A61F 2/30, A61F 2/46, A61F 2/36

(54) **VORRICHTUNG ZUR ERMITTLUNG EINES ABSTANDS, VERFAHREN UND VERWENDUNG**

(71) Anmelder: Heraeus Medical GmbH, 61273 Wehrheim (DE)
(72) Erfinder: Vogt, Sebastian, 61273 Wehrheim (DE); Schmitz, Michael, 61273 Wehrheim (DE); Grün, Harald, 54636 Messerich (DE); Kunkel, Christian, 97828 Marktheidenfeld (DE); Pohlmann, Jochen, 97828 Marktheidenfeld (DE)
(74) Vertreter: Heraeus IP

(57) **Zusammenfassung**

Der Erfindung betrifft eine Vorrichtung zur Ermittlung eines benötigten Abstands eines Kopfbereichs von einem Schaftbereich eines Gelenkspacers, ein System, ein Verfahren zur Herstellung eines Gelenkspacers sowie eine Verwendung. Eine Vorrichtung (10) zur Ermittlung eines benötigten Abstands eines Kopfbereichs (2) von einem Schaftbereich (3) eines Gelenkspacers (1) umfasst einen Kopf (11) und einen Schaft (13), die in unterschiedlichen Abständen zueinander positionierbar sind, wobei die Vorrichtung (10) ferner eine Fixierungseinrichtung (15) zum Fixieren eines Abstands zwischen dem Kopf (11) und dem Schaft (13) aufweist. Die Vorrichtung erlaubt es, die benötigte Gestalt eines Hüftgelenkspacers intraoperativ zu bestimmen, indem der Abstand des Kopfes vom Schaft an die konkrete Anatomie des Patienten angepasst wird.

## Beschreibung

Der Erfindung betrifft eine Vorrichtung zur Ermittlung eines benötigten Abstands eines Kopfbereichs von einem Schaftbereich eines Gelenkspacers, ein System, ein Verfahren zur Herstellung eines Gelenkspacers sowie eine Verwendung. Bevorzugt ist die Vorrichtung außerdem dazu eingerichtet, einen beliebigen Kopf mit einem beliebigen Schaft zu kombinieren, um einen Gelenkspacer zu erhalten, der optimal an die Anatomie des Patienten angepasst ist.

Im Rahmen von zweizeitigen Wechseloperationen von Endoprothesen, beispielsweise Hüft- oder Schultertotalgelenkendoprothesen, werden in der Interimsphase Spacer als temporäre Platzhalter eingesetzt. Ein Einsatz erfolgt insbesondere bei septischen Wechseloperationen. Derartige Spacer werden häufig intraoperativ vom medizinischen Personal hergestellt, beispielsweise aus Knochenzement wie z. B. Polymethylmethacrylat-Knochenzement. Bei der Herstellung dieser Spacer können dem Knochenzement je nach vorliegenden Antibiogramm der für die Infektionen ursächlichen mikrobiellen Keime ein oder mehrere speziell auf die vorliegenden Keime abgestimmte Antibiotika zugesetzt werden.

Es gibt vorgefertigte Spacer, die zwar eine große Stabilität aufweisen, aber nicht an die individuelle Anatomie des Patienten angepasst sind. Deshalb werden bevorzugt individuelle Spacer intraoperativ hergestellt.

Für die intraoperative Herstellung von Spacern, üblicherweise mit Knochenzement, werden üblicherweise Kunststoffgießformen verwendet, wie beispielsweise in der Druckschrift US 6 361 731 B1 beschrieben. Diese Gießformen können mit unterschiedlichen Durchmessern des Spacerkopfes hergestellt werden. Der medizinische Anwender kann dabei zwischen vorgegebenen Größen des Spacerkopfes auswählen. So kann für einen Patienten in Abhängigkeit der konkreten anatomischen Situation ein angepasster Spacer bereitgestellt werden. Ein Spacer für ein Gelenk bzw. ein Gelenkteil wird als Gelenkspacer bezeichnet.

In einer Weiterentwicklung wurden in den Druckschriften US 7 637 729 B2, US 7 789 646 B2, US 8 480 389 B2 und US 8 801 983 B2 mehrteilige Gießformen für die Herstellung modularer Hüftspacer vorgeschlagen. Die Gießformen aus den Schriften US 7 789 646 B2, US 8 480 389 B2 und US 8 801 983 B2 bestehen aus einer Gießform für den Schaft, die mit einer Gießform für den Spacerkopf verbunden werden kann. Es stehen dazu Gießformen für den Spacerkopf mit unterschiedlichen Durchmessern zur Verfügung. Die Gießform des Schafts wird mit der Gießform des Spacerkopfes, die den ausgewählten Durchmesser hat, verbunden. Die so zusammengesetzte Gießform kann dann mit Knochenzement befüllt werden. Nach der Aushärtung wird der gebildete Hüftgelenkspacer entnommen.

In der Druckschrift EP 3 957 280 B1 wird eine Vorrichtung zur Herstellung von Hüftgelenkspacern beschrieben, die es ermöglicht, patientenspezifische Hüftgelenkspacer hinsichtlich der Größe des Spacerkopfes und des Abstands des Kopfes vom Femurschaft (engl.: "femural offset") herzustellen. So wird eine weitere Anpassung des Spacers möglich.

Es ist die Aufgabe der Erfindung, die benötigte Gestalt eines Gelenkspacers einfach und reproduzierbar zu bestimmen, um die individuell angepasste Herstellung von Gelenkspacern zu verbessern.

Die Aufgabe wird gelöst durch die Vorrichtung gemäß Anspruch 1 sowie das System, das Verfahren und die Verwendung gemäß den nebengeordneten Ansprüchen. Vorteilhafte Ausgestaltungen ergeben sich aus den Unteransprüchen.

Zur Lösung der Aufgabe dient eine Vorrichtung zur Ermittlung eines benötigten Abstands eines Kopfbereichs von einem Schaftbereich eines Gelenkspacers. Die Vorrichtung umfasst einen Kopf und einen Schaft, die in unterschiedlichen Abständen zueinander positionierbar sind. Die Vorrichtung weist ferner eine Fixierungseinrichtung zum Fixieren eines Abstands zwischen dem Kopf und dem Schaft auf.

Die Vorrichtung dient dazu, in der Position eines einzusetzenden Spacers in den Körper eines Patienten temporär eingesetzt zu werden, um einen benötigten Abstand des Kopfbereichs des Spacers von dem Schaftbereich des Spacers zu ermitteln bzw. um zu ermitteln, ob ein gewählter Abstand zu den konkreten anatomischen Gegebenheiten passt. Die Vorrichtung ist dazu eingerichtet, den gewählten Abstand zwischen dem Kopf und dem Schaft zu fixieren, um auf diese Weise ein unplanmäßiges Verändern des Abstands oder ein ggf. mögliches, unplanmäßiges Trennen von Schaft und Kopf, das ein Verlieren des Kopfes zur Folge haben könnte, zu verhindern. Insbesondere ist der Abstand während des Einsatzes im Körper des Patienten fixiert. Die Vorrichtung kann auch als Prüflehre bezeichnet werden.

Die Vorrichtung erlaubt es, die benötigte Gestalt eines Hüftgelenkspacers während der Operation zu bestimmen, indem der Abstand des Kopfes vom Schaft (auch Englisch als "femural offset" bezeichnet) an die konkrete Anatomie des Patienten angepasst wird. So kann für den konkreten Patienten individuell der passende Spacer ausgewählt werden. Beispielsweise kann die Herstellung des Spacers dann intraoperativ mit Hüftgelenkspacerformen und/oder gemäß der Druckschrift EP 3 957 280 B1 erfolgen.

Der herzustellende und einzusetzende Spacer umfasst einen Kopfbereich (Kopf) und einen Schaftbereich (Schaft) sowie ggf. einen dazwischen liegenden Halsbereich (Hals), der den Kopfbereich und den Schaftbereich verbindet. Der Spacer ist insbesondere zumindest in seinem Kopfbereich der Form und Größe des entsprechenden Knochens, beispielsweise des Femurs (Oberschenkelknochens), nachempfunden. Der Schaftbereich des Spacers wird in die Öffnung im Knochen eingesetzt und dort typischerweise fixiert. Der Kopfbereich des Spacers ist zumindest bereichsweise näherungsweise kugelförmig, um die bewegliche Verbindung, beispielsweise mit dem Becken, abzubilden, in diesem Fall das Hüftgelenk. Der Spacer kann ferner einen Halsbereich umfassen, der den Schaft mit dem Kopf verbindet.

Auch der Kopf der Vorrichtung ist ähnlich geformt wie der Kopf des entsprechenden Gelenks, beispielsweise des Femurs. Der Kopf kann zumindest bereichsweise näherungsweise eine kugelförmige Außenfläche aufweisen. Der Kopf kann teilweise oder vollständig hohl sein. Der Schaft der Vorrichtung ist dazu eingerichtet, in die Öffnung im Knochen eingesetzt zu werden. Beispielsweise ist der Schaft länglich und verjüngt sich im Querschnitt, um einfach eingesetzt werden zu können.

Der Kopf und der Schaft sind zur Positionierung in unterschiedlichen Abständen typischerweise beweglich zueinander, insbesondere linear verschieblich. Die Fixierungseinrichtung fixiert den Kopf direkt oder indirekt am Schaft. Im festgestellten (fixierten) Zustand sind der Kopf und der Schaft, ggf. mittels eines dazwischen liegenden Halses, so verbunden, dass zumindest eine relative Bewegung zwischen Kopf und Schaft, die den Abstand verändert, verhindert wird. Dies kann etwa eine Bewegung in axialer Richtung sein, bezogen auf eine Längsachse des Schafts oder eines zwischen Schaft und Kopf befindlichen Halses. Eine Längsachse meint insbesondere eine mittlere Längsachse (Mittellängsachse).

In einer einfachen Ausführungsform sind der Schaft und der Kopf mit einem Gewinde verbunden. Beispielsweise weist der Schaft oder ein mit dem Schaft verbundener Hals ein Außengewinde auf und der Kopf weist ein Innengewinde auf. Durch relative Rotation kann dann die axiale Position des Kopfes in Bezug zum Schaft eingestellt werden.

Die Fixierungseinrichtung kann beispielsweise einen Pin enthalten, der beispielsweise herausziehbar ausgestaltet ist, um die Position des Kopfes in Bezug zum Schaft fixieren kann. Der Pin kann dazu z. B. in eine geeignete Aufnahme bewegt werden. Alternativ oder ergänzend kann die Fixierungseinrichtung ein Rastelement aufweisen, das mit einer Feder vorgespannt sein kann. Auf diese Weise kann in einem eingerasteten und mit der Feder gesicherten Zustand eine Bewegung zwischen Kopf und Schaft gesperrt werden. Wird das Rastelement gegen die Federkraft gelöst, beispielsweise durch manuelles Drücken, kann eine Bewegung zwischen Kopf und Schaft freigegeben werden. Es kann vorgesehen sein, dass die Bewegung nur im gedrückten Zustand des Rastelements möglich ist.

In einer Ausgestaltung weist die Vorrichtung ferner einen Hals auf, der den Kopf mit dem Schaft verbindet. Der Hals ist insbesondere fest mit dem Schaft verbunden. Der Hals kann einstückig mit dem Schaft sein. Insbesondere ist der Kopf dazu eingerichtet, auf den Hals geschoben oder geschraubt zu werden und/oder am Hals fixiert zu werden.

Insbesondere ist der Hals so eingerichtet, dass zwischen dem Schaft und dem Kopf unterschiedliche Abstände einstellbar sind. Beispielsweise kann der Kopf in unterschiedlichen Positionen am Hals angeordnet werden.

Der Hals kann einen Winkel mit dem Schaft einschließen, der nicht 180° beträgt. Der Winkel kann im Bereich des Femurs dem CCD-Winkel (Centrum-Collum-Diaphysen-Winkel) entsprechen. Der Winkel kann mindestens 100°, bevorzugt mindestens 110°, insbesondere mindestens 120° und/oder höchstens 160°, bevorzugt höchstens 150°, insbesondere höchstens 140° betragen. Diese Ausgestaltung ermöglicht die Herstellung eines besonders gut angepassten Spacers, der an die Anatomie des Patienten optimal angepasst ist.

In einer Ausgestaltung liegen der Kopf einerseits und der Schaft und/oder der Hals andererseits getrennt voneinander vor oder können voneinander getrennt werden.

In dieser Ausgestaltung können der Schaft und der Kopf, ggf. indirekt über den Hals, miteinander verbunden und dann in der gewünschten Position mittels der

Fixierungseinrichtung relativ zueinander fixiert werden. Dies ermöglicht es, den Kopf oder den Schaft auszutauschen bzw. gezielt zu wählen, um unterschiedliche Kombinationen aus Schaft und Kopf zu verwenden. Beispielsweise kann eine gewünschte Größe des Kopfes gewählt werden, die am besten zur Anatomie des Patienten passt. Alternativ oder ergänzend kann ein Schaft mit einer bestimmten Länge und/oder einem bestimmten Durchmesser gewählt werden, der am besten zur Anatomie des Patienten passt.

In einer Ausgestaltung ist der Kopf auf dem Hals verschieblich. Die Fixierungseinrichtung weist mindestens einen Stift sowie eine Vielzahl an Formschlusselementen auf. Der Stift kann so mit einem oder zwei Formschlusselementen in Kontakt gebracht werden, dass ein Verschieben des Kopfes auf dem Hals gesperrt ist.

Der Stift ist an einem der zwei relativ zueinander verschieblichen Teile angeordnet, insbesondere an dem Kopf. Die Formschlusselemente sind an dem anderen der zwei relativ zueinander verschieblichen Teile angeordnet, insbesondere an dem Hals. Insbesondere ist der Kopf auf dem Hals entlang der axialen Richtung bezogen auf die Längsachse des Halses verschieblich. Die Längsachse des Halses kann der Symmetrieachse und/oder Längsachse des Kopfes entsprechen. Insbesondere wird die axiale Position des Kopfes auf dem Hals fixiert.

Der Stift ist ein gegenüber seiner Umgebung herausragendes Element. Der Stift kann einen runden oder eckigen, beispielsweise auch länglichen bzw. rechteckigen Querschnitt aufweisen. Die Form des Stifts kann grundsätzlich beliebig sein. Es ist lediglich notwendig, dass der Stift mit dem Formschlusselement in Kontakt gebracht werden kann, um das Verschieben zu sperren. Insbesondere ist der Stift zumindest ungefähr in radialer Richtung ausgerichtet.

Der Stift kann typischerweise mit jedem der Formschlusselemente in Kontakt gebracht werden. Die Formschlusselemente sind typischerweise in unterschiedlichen Positionen in Bezug zu einer Längsachse desjenigen Elements, das die Formschlusselemente aufweist, angeordnet, insbesondere in einer Reihe. Durch die Wahl des mit dem Stift in Kontakt zu bringenden Formschlusselements wird so die relative Position und damit der Abstand zwischen Kopf und Hals fixiert. Die Formschlusselemente sind insbesondere in Umfangsrichtung ausgerichtet.

Grundsätzlich reicht ein Kontakt des Stifts mit einem Formschlusselement, um ein Verschieben in einer Richtung zu blockieren. Bevorzugt kann der Stift zwischen zwei Formschlusselementen angeordnet werden, um so ein Verschieben in beide Richtungen zu blockieren. Zwischen zwei benachbarten Formschlusselementen befindet sich insbesondere jeweils ein Zwischenraum, in dem der Stift positioniert werden kann, um den Abstand zu fixieren.

Grundsätzlich sind mindestens drei Formschlusselemente und zwei dazwischen liegende Zwischenräume vorhanden, so dass je nach Wahl des Zwischenraums zwei verschiedene Abstände eingestellt werden können. Insbesondere sind mindestens vier oder fünf, oder mehr Formschlusselemente vorhanden.

Insbesondere sind zwei Stifte vorhanden, die in axialer Richtung voneinander beabstandet sind. Typischerweise liegen die zwei Stifte auf einer gedachten Linie, die in axialer Richtung verläuft. Die axiale Richtung bezieht sich auf die Komponente, die die Stifte aufweist. Auf diese Weise wird ein Verkanten sicher verhindert. Es können zwei Sätze von Formschlusselementen und dazwischen befindlichen Zwischenräumen vorhanden sein. Jeder Stift kann dann in einen Satz von Formschlusselementen eingreifen.

In einer Ausgestaltung ist angrenzend an die Vielzahl an Formschlusselementen eine axial ausgerichtete Nut angeordnet. Der Stift kann in der Nut in axialer Richtung verschoben werden. So kann der Abstand eingestellt werden.

Hierbei sind die Formschlusselemente insbesondere kammartig ausgebildet. Die an die Formschlusselemente angrenzende, axial ausgerichtete Nut, dient zum Verschieben insbesondere des Kopfes auf dem Hals. Der insbesondere am Kopf angeordnete Stift kann in der Nut gleiten, während der Kopf axial auf dem Hals verschoben wird. In Draufsicht sind die Zwischenräume und/oder die Formschlusselemente senkrecht zur Nut angeordnet.

Insbesondere kann dann durch eine Drehung des Kopfes relativ zum Hals um eine axiale Achse ein Eingriff hergestellt werden, in dem der Stift zwischen zwei Formschlusselementen angeordnet wird und eine axiale Verschiebung verhindert wird. Ein eingestellter Abstand kann demnach nach dem Einstellen des gewünschten Abstands durch eine relative Drehung des Kopfs in Bezug zum Hals fixiert werden. Insbesondere ist die Nut in Bezug auf eine Umfangsfläche des die Nut aufweisenden Bauteils in einer benachbarten Winkelposition des Bauteils zu den Formschlusselementen bzw. deren Zwischenräumen angeordnet. Die Nut ist insbesondere direkt mit den jeweiligen Zwischenräumen verbunden, so dass bei einer relativen Drehung der Stift von der Nut in einen der Zwischenräume bewegt werden kann und auf diese Weise der Abstand fixiert werden kann.

In einer Ausgestaltung umfasst die Fixierungseinrichtung eine Umschalteinrichtung zum Umschalten von einer Öffnungsposition in eine Schließposition. Insbesondere kann in der Öffnungsposition der Abstand zwischen Kopf und Schaft verändert werden und/oder ist in der Schließposition der Abstand zwischen Kopf und Schaft fixiert.

In der Öffnungsposition kann demnach ein gewünschter Abstand eingestellt werden. Anschließend kann umgeschaltet werden, um den gewünschten Abstand zu fixieren. Insbesondere ist die Umschalteinrichtung ebenso dazu eingerichtet, von der Schließposition zurück in die Öffnungsposition zu schalten. Bevorzugt kann mehrmals zwischen den zwei Positionen hin- und hergeschaltet werden. Auf diese Weise kann mit mehreren Iterationen der gewünschte Abstand ermittelt werden.

Die Umschalteinrichtung kann insbesondere manuell betätigt werden. Bevorzugt ist die Umschalteinrichtung eine mechanische Umschalteinrichtung. In diesem Fall werden für das Umschalten keine elektrischen oder elektronischen Bauteile benötigt.

Grundsätzlich kann das Umschalten durch Bewegen einer Komponente der Vorrichtung relativ zu einer anderen Komponente der Vorrichtung erfolgen, beispielsweise durch Bewegen eines Schalters in Bezug zu einem Gehäuseteil. Das Bewegen kann eine lineare und/oder eine rotatorische Bewegung sein.

Das Verändern des Abstands zwischen Kopf und Schaft in der Öffnungsposition muss nicht notwendigerweise durch alleiniges Verschieben möglich sein. Es kann notwendig sein, vor dem Verschieben eine Drehung durchzuführen, um beispielsweise einen Stift außer Eingriffs mit einem oder mehreren Formschlusselementen zu bringen. Insbesondere ist in der Öffnungsposition eine Bewegung zwischen Kopf und Schaft möglich.

Diese Ausgestaltung verhindert eine unerwünschte Verschiebung des Kopfes gegenüber dem Hals und ein Lösen des Kopfs, etwa während des Prüfens der möglichen Gestalt des Spacers mit der Vorrichtung im Patienten.

In einer Ausführungsform umfasst die Vorrichtung eine Skala zum Ablesen eines Abstands zwischen Kopf und Schaft. Die Skala kann im Bereich von Formschlusselementen angeordnet sein, so dass die Position eines Stifts eine Position bzw. einen Abstand auf der Skala anzeigt.

In einer Ausgestaltung erfolgt das Umschalten durch Rotation einer rotierenden Einheit in Bezug zu einem Gehäuseteil um eine Längsachse des Halses. Eine rotierende Einheit ist eine Einheit, die drehbar gelagert ist. Insbesondere kann das Umschalten zurück in die andere Position dann durch erneute Rotation, beispielsweise in die entgegengesetzte Richtung, erfolgen.

Insbesondere weist der Hals ein Gehäuse auf. Das Gehäuse kann die äußere Hülle des Halses definieren. Das Gehäuse stellt einen Übergang zwischen dem Kopf, beispielsweise verschiedenen Köpfen, und dem Schaft, beispielsweise verschiedenen Schäften dar, und kann daher auch als Adapter bezeichnet werden. Insbesondere ist die rotierende Einheit gegenüber dem gesamten Gehäuse drehbar.

In einer Ausgestaltung ist ein Sperrelement vorhanden, welches beim Umschalten in die Schließposition aktiviert wird und jede Bewegung zwischen Kopf und Schaft blockiert. In dieser Ausgestaltung ist in der Schließposition sowohl eine axiale Verschiebung als auch ein Rotation zwischen Kopf und Hals gesperrt. Es kann so auch ein versehentliches Lösen verhindert werden. In der Schließposition ist daher keine Bewegung zwischen Kopf und Schaft möglich.

Wenn das Rotieren durch Rotation einer rotierenden Einheit in Bezug zu einem Gehäuseteil erfolgt und wenn die rotierende Einheit, kann das Sperrelement Teil der rotierenden Einheit sein. So kann das Sperrelement durch Rotation auf einfache Weise aktiviert und deaktiviert werden. Das Sperrelement ist insbesondere Teil der Umschalteinrichtung.

Das Sperrelement kann in der Schließposition ein Bewegen des Stifts aus einem Zwischenraum zwischen zwei Formschlusselementen in Umfangsrichtung verhindern. Dies kann ein Drehen der rotierbaren Einheit im Hals bzw. im Gehäuse des Halses verhindern. Das Sperrelement kann ein axial verlaufender Steg sein. Es kann in radialer Richtung neben dem Steg eine axial verlaufende Nut oder Aussparung vorhanden sein, so dass in der Öffnungsposition ein Bewegen des Stifts aus dem Zwischenraum heraus möglich ist.

Es kann eine fensterartige Öffnung vorhanden sein, beispielsweise im Gehäuse des Halses, durch welche das Sperrelement aus einer versenkten oder Öffnungsposition in die Schließposition bewegt, beispielsweise gedreht, werden kann.

Beispielsweise kann nach dem Einstellen und Fixieren eines gewünschten Abstands wie oben beschrieben das Sperrelement aktiviert werden, um den gewünschten Abstand zu sichern.

In einer Ausgestaltung ist das Sperrelement mit einem federnd gelagerten Knopf verbunden, welcher mit zwei Aussparungen zusammenwirkt. Der Knopf ist in einer ersten Aussparung positioniert, wenn sich die Fixierungseinrichtung in der Schließposition befindet. Der Knopf ist in einer zweiten Aussparung positioniert, wenn sich die Fixierungseinrichtung in der Öffnungsposition befindet.

Die Positionierung des Knopfs zeigt also die Schließposition bzw. die Öffnungsposition an. Der Knopf ist insbesondere an der rotierenden Einheit angeordnet. Die Aussparungen befinden sich insbesondere im Gehäuseteil. Der Knopf ist in der Aussparung typischerweise so gesichert, etwa durch Formschluss, dass ein Umschalten nicht möglich ist. Insbesondere ist ein Drehen der rotierenden Einheit gegenüber dem Gehäuseteil deshalb nicht möglich, weil der Knopf so in der Aussparung angeordnet ist, dass ein Drehen der rotierenden Einheit gegenüber dem Gehäuseteil gesperrt ist. Für ein Umschalten kann der Knopf in radialer Richtung einwärts gedrückt werden, so dass er gegen die Federkraft unter das die Aussparung ausbildende Material taucht. In dieser Position ist ein Drehen des rotierbaren Teils relativ zum Gehäuseteil möglich. Befindet sich der Knopf dann unter der anderen Aussparung, wird er durch die Federkraft in radialer Richtung auswärts gedrückt und rastet so in die andere Aussparung ein. Nun befindet sich die Vorrichtung in der jeweils anderen Position.

Die zwei Aussparungen sind insbesondere fensterartig ausgebildet und können deshalb auch als Fenster bezeichnet werden. Die zwei Aussparungen können verbunden sein. Es kann sich also um zwei unterschiedliche, definierte Bereiche einer einzigen Aussparung handeln, die erfindungsgemäß als zwei Aussparungen bezeichnet werden. Die zwei Aussparungen sind insbesondere an unterschiedlichen Winkelpositionen in Bezug zur Längsachse angeordnet. Die zwei Aussparungen sind insbesondere an derselben Längenposition in Bezug zur Längsachse angeordnet.

Der Knopf ist Teil des Sperrelements und insbesondere fest mit diesem verbunden. Sperrelement und Knopf rotieren demnach zusammen um die Längsachse. Der Knopf ist insbesondere an einer blattförmigen Feder befestigt und nach außen vorgespannt. Neben den Aussparungen können z. B. visuelle Symbole angeordnet sein, die die jeweilige Position (Öffnungsposition/ Schließposition) anzeigen, so dass die aktuelle Position anhand der Position des Knopfes direkt ersichtlich ist.

In einer Ausgestaltung weist die Fixierungseinrichtung ferner mindestens einen weiteren Stift auf, der sich ausgehend von dem Stift in einer um 180° um die Längsachse gedrehten Position befindet. Alternativ oder ergänzend weist die Fixierungseinrichtung ferner eine weitere Vielzahl an Formschlusselementen auf, die sich ausgehend von der Vielzahl an Formschlusselementen in einer um 180° um die Längsachse gedrehten Position befindet.

Die Fixierung gegen die axiale Bewegung erfolgt also an zwei gegenüberliegenden Positionen. So kann ein Verkanten in Bezug zur Längsachse verhindert und eine sichere Fixierung sichergestellt werden. Es kann in jeder Winkelposition zwei Stege und zwei Sätze von Formschlusselementen geben, um ein Verkanten senkrecht zur Längsachse zu verhindern.

In einer Ausgestaltung weist die Vorrichtung im Bereich des Kopfes und/oder des Schafts eine äußere Hülle auf, die einen Hohlraum nach außen hin begrenzt. Insbesondere ist in der äußeren Hülle eine Einfüllöffnung zum Einfüllen von Knochenzement in den Hohlraum angeordnet.

Die äußere Hülle ist typischerweise nicht vollständig hohl. Der Hohlraum erstreckt sich insbesondere zwischen der äußeren Hülle und einem Kern des Kopfs bzw. des Schafts Vorrichtung, der beispielsweise aus Metall sein kann. Der Kern kann der Stabilisierung und/oder Einstellung der Vorrichtung dienen. Insbesondere ist der Kopf und/oder der Schaft zumindest teilweise hohl. Der Hals kann ebenfalls teilweise hohl ausgebildet sein. Die Vorrichtung kann im Schaft und/oder im Hals eine zusätzliche Metallverstärkung enthalten.

Die Einfüllöffnung dient dem Einfüllen von Knochenzement. Die Einfüllöffnung ist typischerweise dazu eingerichtet, jeden beliebigen Füllstoff einzufüllen. Knochenzement kann dann im Inneren der Vorrichtung aushärten und so einen feste und stabile Spacer ausbilden.

Insbesondere kann die Vorrichtung dabei als verlorene Form dienen. Die äußere Hülle verbleibt dann an dem Knochenzement und bildet gemeinsam mit diesem und ggf. einem oder mehreren Kernen, z. B. aus Metall, den Spacer aus. So kann aus der Vorrichtung direkt ein formstabiler Spacer hergestellt werden. Typischerweise ist mindestens eine Einfüllöffnung im Kopf und mindestes eine Einfüllöffnung im Schaft vorhanden.

Es ist besonders bevorzugt, dass zumindest die äußere Hülle der Vorrichtung aus Polymethylmethacrylat hergestellt ist. Im zum Schaft weisenden Ende des Halses kann ein Durchlass vorhanden sein, durch den Knochenzement, der in den Schaft eingefüllt worden ist, ins Innere des Halses strömen kann.

Typischerweise ist zumindest an einer der Einfüllöffnung abgewandten Seite des jeweiligen Hohlraums im Schaft und/oder im Kopf zumindest eine Entlüftungsöffnung angeordnet. Auf diese Weise kann beim Einfüllen des Knochenzements die im Hohlraum befindliche Luft auf einfache Weise ausströmen, so dass keine Luftblasen im Hohlraum verbleiben.

In einer Ausgestaltung umfasst die Vorrichtung ein biokompatibles Material oder ist aus einem solchen Material hergestellt. Beispielsweise kann biokompatibler Kunststoff verwendet werden. Biokompatibel meint die Eigenschaft, im direkten Kontakt mit lebenden Geweben keinen negativen Einfluss auf deren Stoffwechsel ausüben.

In einer Ausgestaltung kann die Vorrichtung einen oder mehrere der folgenden Stoffe umfassen oder daraus hergestellt sein: Polycarbonat, Polyethylen, Polypropylen, Polyethylenterephthalat, Polybutylenterephthalat, Polymethylmethacrylat.

In einer Ausgestaltung ist die Vorrichtung sterilisierbar. Insbesondere ist die Vorrichtung mittels Gammastrahlung, Elektronenstrahlung, Röntgenstrahlung und/oder Ethylenoxid sterilisierbar.

Ein weiterer Aspekt der Erfindung ist ein System, umfassend eine insbesondere erfindungsgemäße Vorrichtung sowie
- mindestens einen zusätzlichen Kopf, der an dem Schaft der Vorrichtung positionierbar ist, wobei der Kopf eine andere Größe aufweist als der Kopf der Vorrichtung, und/oder
- mindestens einen zusätzlichen Schaft, an dem der Kopf der Vorrichtung positionierbar ist, wobei der Schaft eine andere Größe aufweist als der Schaft der Vorrichtung.

Unterschiedliche Größen von Köpfen beziehen sich insbesondere auf deren Durchmesser. Unterschiedliche Größen von Schäften beziehen sich insbesondere auf deren axiale Länge und/oder Durchmesser.

Insbesondere sind mindestens zwei zusätzliche Köpfe in jeweils unterschiedlichen Größen vorhanden. Ggf. sind mindestens zwei zusätzliche Schäfte in jeweils unterschiedlichen Größen vorhanden.

In einer Ausführungsform umfasst das System mindestens drei zusätzliche Köpfe und mindestens drei zusätzliche Schäfte. Es können z. B. insgesamt vier lange Schäfte und vier kurze Schäfte vorhanden sein. Mit drei oder vier Raststufen zur Abstandseinstellung sind auf diese Weise zig bis Hunderte Kombinationen möglich. Das System kann als Kit verpackt sein und dem medizinischen Personal auf diese Weise für eine Operation zur Verfügung gestellt werden.

Ein weiterer Aspekt der Erfindung ist ein Verfahren zur Ermittlung eines benötigten Abstands eines Kopfbereichs von einem Schaftbereich eines Gelenkspacers, bei dem ein Kopf und ein Schaft einer Vorrichtung in einem gewünschten Abstand zueinander positioniert werden und der Abstand zwischen dem Kopf und dem Schaft fixiert wird. Die Vorrichtung kann eine erfindungsgemäße Vorrichtung sein. Alle Merkmale, Ausgestaltungen und Vorteile der eingangs beschriebenen Vorrichtung können auch für das Verfahren gelten und umgekehrt.

Ein weiterer Aspekt ist ein Verfahren zur Herstellung eines Gelenkspacers, bei dem ein Kopf und ein Schaft einer Vorrichtung in einem gewünschten Abstand zueinander positioniert werden, der Abstand fixiert wird und Knochenzement in den Kopf und/oder den Schaft gefüllt wird.

Die Vorrichtung kann eine erfindungsgemäße Vorrichtung sein. Alle Merkmale, Ausgestaltungen und Vorteile der eingangs beschriebenen Vorrichtung können auch für das Verfahren gelten und umgekehrt.

Zunächst wird mittels Positionieren von Kopf und Schaft und Fixieren ein benötigter Abstand ermittelt. Anschließend wird die Vorrichtung selbst verwendet, um den Gelenkspacer herzustellen. Die Vorrichtung wird hierbei als Gießform verwendet. Der Knochenzement härtet anschließend in der Vorrichtung aus. Der Knochenzement ist insbesondere Polymethylmethacrylat-(PMMA-)Knochenzement.

Das Verfahren kann einen oder mehrere der folgenden Schritte in beliebiger Kombination umfassen:
- Verbinden von Kopf und Hals,
- Verschieben des Kopfes auf dem Hals, um den benötigten Abstand einzustellen,
- Sperren des Verschiebens durch Inkontaktbringen eines Stifts mit einem oder zwei Formschlusselementen und/oder durch Rotation des Kopfes gegenüber dem Hals um eine Längsachse,
- Umschalten von einer Öffnungsposition in eine Schließposition, um den Abstand zwischen Kopf und Schaft zu fixieren und/oder eine Rotation des Kopfes gegenüber dem Hals zu sperren, insbesondere durch Rotation einer rotierenden Einheit, wobei insbesondere ein Knopf aus einer Aussparung gedrückt wird.

Insbesondere wird ein jeweiliger Hohlraum im Kopf und/oder im Schaft zumindest teilweise mit Knochenzement gefüllt. Dies erfolgt insbesondere über eine Einfüllöffnung.

In einer Ausgestaltung bildet eine äußere Hülle der Vorrichtung eine Außenhülle des Spacers. Mit anderen Worten definiert die äußere Hülle der Vorrichtung nach dem Aushärten des Knochenzements die Außenhülle des Spacers oder dient als Außenhülle des Spacers. Die Vorrichtung wird also als verlorene Schalung zur Herstellung des Spacers verwendet.

Ein weiterer Aspekt der Erfindung ist eine Verwendung einer verlorenen Schalung zur Herstellung des Gelenkspacers.

Insbesondere wird eine Vorrichtung zur Ermittlung eines benötigten Abstands eines Kopfbereichs von einem Schaftbereich eines Gelenkspacers als verlorene Schalung zur Herstellung des Gelenkspacers verwendet. Die Vorrichtung kann eine erfindungsgemäße Vorrichtung sein.

Eine verlorene Schalung ist eine Schalung, die nach der Herstellung des zu gießenden Teils an Ort und Stelle verbleibt und damit Teil des hergestellten Teils wird. Nachfolgend werden Ausführungsbeispiele der Erfindung auch anhand von Figuren näher erläutert. Merkmale der Ausführungsbeispiele können einzeln oder in einer Mehrzahl mit den beanspruchten Gegenständen kombiniert werden, sofern nichts Gegenteiliges angegeben ist. Die beanspruchten Schutzbereiche sind nicht auf die Ausführungsbeispiele beschränkt.

Es zeigen:
- Figur 1:: eine perspektivische Darstellung einer Vorrichtung,
- Figuren 2 bis 4:: Schritte der Verwendung einer Vorrichtung,
- Figur 5:: eine Explosionszeichnung einer Vorrichtung,
- Figur 6:: eine rotierende Einheit einer Vorrichtung, sowie
- Figuren 7 bis 9:: Schritte der Verwendung einer Vorrichtung.

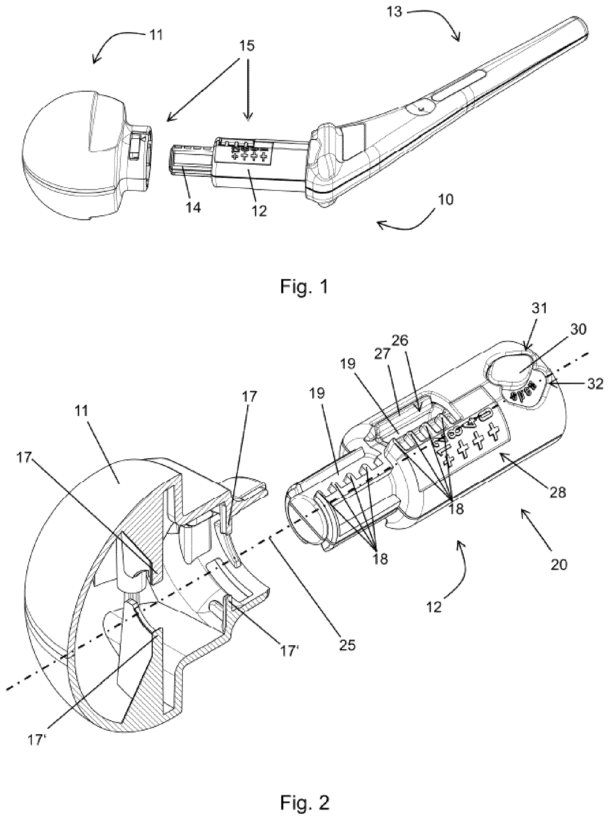

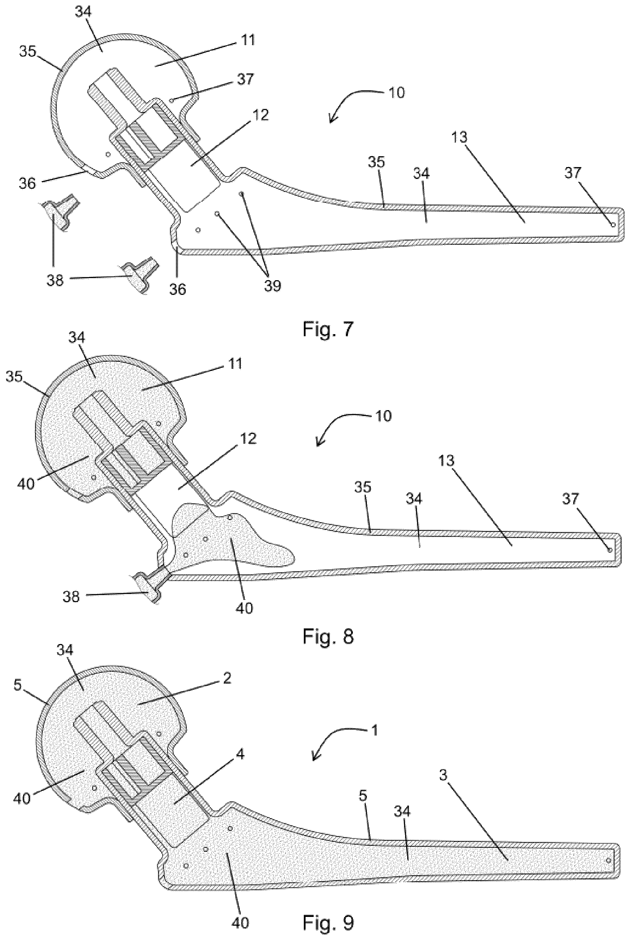

Figur 1 zeigt eine Vorrichtung 10 zur Ermittlung eines benötigten Abstands eines Kopfbereichs von einem Schaftbereich eines Gelenkspacers. Ein Gelenkspacer 1 ist beispielhaft in Figur 9 dargestellt.

Die Vorrichtung 10 umfasst einen Kopf 11 und einen Schaft 13. Grundsätzlich können Kopf 11 und Schaft 13 in unterschiedlichen Abständen zueinander angeordnet werden. Fest in einem Winkel am Schaft 13 angeordnet befindet sich ein Hals 12 der Vorrichtung. Der Hals 12 kann an seinem freien Ende einen vom Schaft 13 weg weisenden Fortsatz 14 aufweisen. Der Fortsatz 14 kann im Vergleich zum Schaft 13 einen reduzierten Durchmesser aufweisen. Der Kopf 11 kann im hier gezeigten Beispiel auf den Hals 12, insbesondere auf dessen Fortsatz 14, aufgesteckt werden. Dabei kann der Fortsatz 14 in einer gewünschten Tiefe in den Kopf 11 eintauchen.

Ferner umfasst die Vorrichtung eine Fixierungseinrichtung 15, mit der der Abstand zwischen Kopf 11 und Schaft 13 fixiert werden kann. Im hier gezeigten Ausführungsbeispiel sind Teile der Fixierungseinrichtung 15 am bzw. im Kopf 11 angeordnet und Teile der Fixierungseinrichtung 15 am Hals 12 angeordnet. Die hier gezeigte Fixierungseinrichtung 15 ist demnach dazu eingerichtet, die relative Position zwischen Kopf 11 und Hals 12 zu fixieren.

Details der Fixierung mittels der Fixierungseinrichtung 15 sind in den Figuren 2 bis 4 dargestellt. Figur 2 zeigt eine Schnittdarstellung des Kopfes 11 und eine perspektivische Darstellung des Halses 12. Hals 12 und Kopf 11 liegen separat voneinander vor und sind auf einer gemeinsamen Achse angeordnet, nämlich der Längsachse 25 des Halses 12. Der Kopf 11 kann entlang dieser Achse auf den Hals 12 geschoben werden und dort fixiert werden. Es ist eine Skala 28 vorhanden, auf der die relative Position des Kopfes 11 zum Hals 12 bzw. der Abstand zwischen Kopf 11 und Schaft 13 abgelesen werden kann.

Am bzw. im Kopf 11 befinden sich zwei Stifte 17, die axial voneinander beabstandet sind. Insbesondere ist zu jedem Stift 17 ein weiterer Stift 17' vorhanden, der insbesondere eine um 180° um die Längsachse 25 gedrehte Kopie des jeweiligen Stifts 17 ist. Am Hals 12 befinden sich zwei Sätze aus Formschlusselementen 18. Die Formschlusselemente 18 sind als parallele Stege ausgebildet, die entlang der Umfangsrichtung des Halses 12 an dessen Oberfläche verlaufen. Zwischen benachbarten Formschlusselementen 18 sind jeweilige Zwischenräume angeordnet. in Umfangsrichtung neben den Formschlusselementen 18 ist eine in axialer Richtung verlaufende Nut 19 angeordnet, die die Zwischenräume miteinander verbindet. An der nicht sichtbaren Unterseite sind insbesondere zwei Sätze weiterer Formschlusselemente 18'. Diese sind jeweils eine um 180° die Längsachse 25 gedrehte Kopie der jeweiligen Sätze von Formschlusselementen 18.

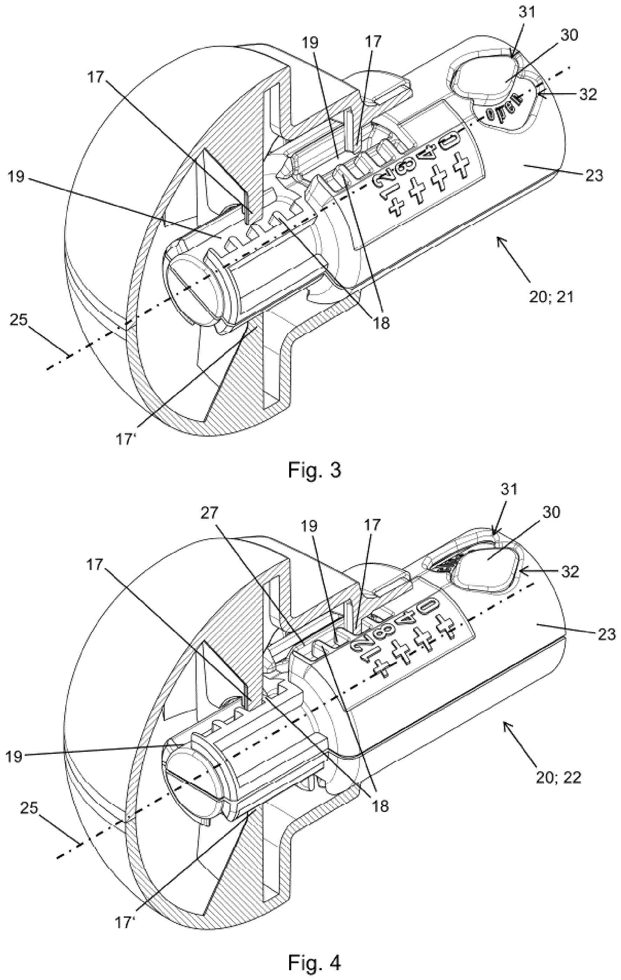

Wird der Kopf 11 auf den Hals 12 aufgeschoben, bewegt sich jeder Stift 17 und falls vorhanden jeder weitere Stift 17' in axialer Richtung in einer Nut 19. Eine derartige Position ist in Figur 3 dargestellt.

Erfolgt anschließend eine Drehung des Kopfes 11 in Uhrzeigerrichtung gegenüber dem Hals 12, werden die Stifte 17 und die weiteren Stifte 17` in einen der Zwischenräume bewegt und werden von den angrenzenden Formschlusselementen 18 bzw. weiteren Formschlusselementen 18' in gegen ein Verschieben in axialer Richtung gehalten. Auf diese Weise wird der Abstand zwischen Kopf 11 und Hals 12 und damit auch zwischen Kopf 11 und Schaft 13 fixiert.

Um ein versehentliches Lösen dieser Fixierung zu lösen, ist ein Sperrelement 27 vorhanden, das in den Bereich der Nut bewegt werden kann. Auf diese Weise kann eine Bewegung des jeweiligen Stifts 17 oder weiteren Stifts 17` aus dem Zwischenraum heraus in die Nut 19 verhindert werden. Das Sperrelement 37 ist Teil einer Umschalteinrichtung 20, die dazu eingerichtet ist, von der in Figur 3 gezeigten Öffnungsposition 21 in die in Figur 4 gezeigte Schließposition 22 umzuschalten. In der Öffnungsposition 21 kann durch relative Rotation zwischen Kopf 11 und Hals 12 der Stift 17 und ggf. der weitere Stift 17' in die Nut 19 bewegt werden und in der Folge kann der Kopf 11 in Bezug zum Schaft 13 axial bewegt werden. Somit kann der Abstand zwischen Kopf 11 und Schaft 13 verändert werden. In der Schließposition 22 ist dagegen die Bewegung des Stifts 17 und ggf. des weiteren Stifts 17` aus dem Zwischenraum heraus gesperrt. Somit ist jede Bewegung zwischen Kopf 11 und Schaft 13 blockiert. Der Abstand zwischen Kopf 11 und Schaft 13 ist fixiert.

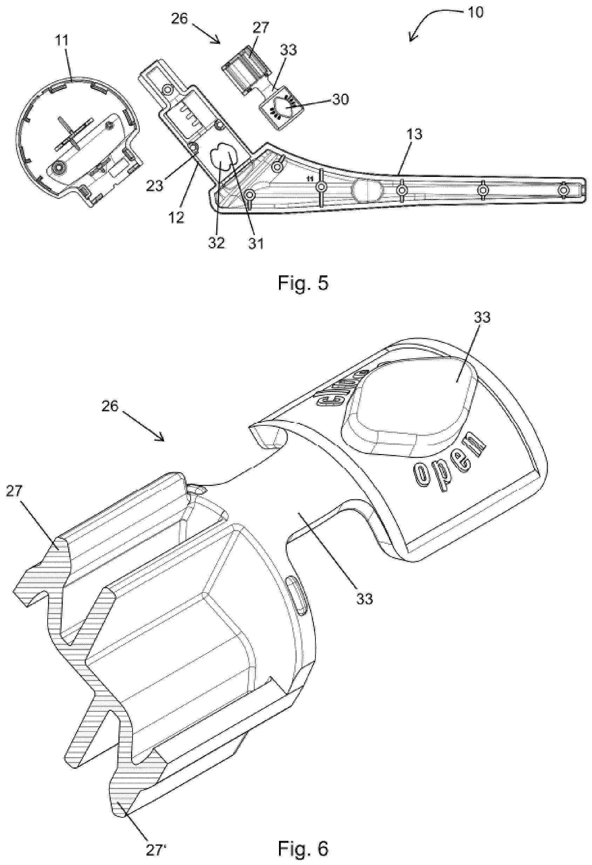

Im hier dargestellten Ausführungsbeispiel ist das Sperrelement 27 Teil einer rotierenden Einheit 26, die im Inneren des Halses 12 angeordnet und relativ zu einem äußeren Gehäuseteil 23 des Halses 12 drehbar ist. Dies ist näher in Bezug zu den Figuren 5 und 6 erläutert. Die rotierende Einheit kann ein weiteres Sperrelement 27' umfassen, welches eine um 180° um die Längsachse 25 gedrehte Kopie des Sperrelements 27 ist (siehe Figur 6).

Figur 5 zeigt eine Explosionszeichnung der beispielhaften Vorrichtung 10. Neben den bereits beschriebenen Komponenten Kopf 11, Hals 12 und Schaft 13 ist die rotierende Einheit 26 erkennbar, die Teil der Umschalteinrichtung 20 ist und an der sich das Sperrelement 17 befindet. Die rotierende Einheit 26 umfasst ferner einen Knopf 30, der über ein Federelement 33 mit dem Rest der rotierenden Einheit 26 verbunden ist. An einem Gehäuseteil 23 des Halses 12, innerhalb dessen die rotierende Einheit 26 drehbar angeordnet ist, sind zwei Aussparungen 31, 32 vorhanden, die jeweils dazu eingerichtet sind, den Knopf 30 aufzunehmen. Die Aussparungen 31, 32 sind benachbart und im gezeigten Ausführungsbeispiel miteinander verbunden. Ist der Knopf 30 in der ersten Aussparung 31 angeordnet, befindet sich die Vorrichtung 10 in der Schließposition 22. Ist der Knopf 30 in der zweiten Aussparung 33 angeordnet, befindet sich die Vorrichtung 10 in der Öffnungsposition 21.

Der Knopf 30 kann entgegen der Federkraft radial nach innen gedrückt werden. So kann der Knopf 30 aus einer Aussparung 31, 32 gelöst werden. Nun kann die rotierende Einheit 26 gegenüber dem Gehäuseteil 23 rotiert werden, bis der Knopf 30 die andere Aussparung 32, 31 erreicht und dort durch die Federkraft radial nach außen in die Aussparung hinein schnappt. Durch die Anordnung in der jeweiligen Aussparung ist einerseits die jeweilige Öffnungsposition bzw. Schließposition fixiert und andererseits ist einfach ersichtlich, in welcher Position sich die Fixierungseinrichtung gerade befindet. Zu diesem Zweck sind im Bereich der Aussparungen 31, 32 und/oder des Knopfes 30 typischerweise entsprechende Beschriftungen vorgesehen, wie in den Figuren 3 bis 6 beispielhaft dargestellt.

Figur 6 zeigt eine vergrößerte, teilgeschnittene Darstellung der rotierenden Einheit 26, in der der Knopf 30 sowie das Federelement 33 erkennbar sind. Das Federelement ist beispielhaft ein blatt- bzw. zungenförmiges Teil, das z. B. aus Kunststoff hergestellt sein kann, beispielsweise einstückig mit dem Knopf und/oder der gesamten rotierenden Einheit 26. Im links gezeigten Schnittbild ist das radial nach außen ragende Sperrelement 27 sowie ein entsprechend ausgebildetes weiteres Sperrelement 27' erkennbar.

Die Figuren 7 bis 9 zeigen weitere Aspekte der Erfindung. Die Vorrichtung 10 gemäß Figur 7 umfasst eine äußere Hülle 35, die einen inneren Hohlraum 34 nach außen hin begrenzt, und zwar insbesondere sowohl im Bereich des Kopfes 11 als auch des Schafts 13. Mit anderen Worten sind der Kopf 11 und der Schaft 13 zumindest teilweise hohl. Die äußere Hülle weist Einfüllöffnungen 36 auf, durch die Knochenzement oder ein anderes geeignetes Material eingefüllt werden können. Die Vorrichtung 10 weist in der äußeren Hülle 35 ferner Entlüftungsöffnungen 37 auf. Diese sind bevorzugt so angeordnet, dass beim Einfüllen von Knochenzement 40, etwa mit einem oder mehreren Applikatoren 38, wie in Figur 8 gezeigt, die verdrängte Luft ausströmen kann. Ein Hohlraum 34 des Halses 12 ist insbesondere mit einem Hohlraum 34 des Kopfes 11 und/oder einem Hohlraum 34 des Schafts 13 verbunden, damit dort indirekt, über den jeweils verbundenen Teil, Knochenzement 40 einströmen kann.

Figur 9 zeigt die Situation nach dem vollständigen Einfüllen und Aushärten des Knochenzements 40. Aus der Vorrichtung 10 ist nun ein Gelenkspacer 1 geworden. Der Gelenkspacer 1 ist individuell an die Anatomie des Patienten angepasst. Der Kopf 11, der Hals 12 und der Schaft 13 der Vorrichtung 10 sind zu einem Kopfbereich 2, einem Halsbereich 4 und einem Schaftbereich 3 des Gelenkspacers 1 geworden. Der Gelenkspacer ist hergestellt und besteht aus der Vorrichtung 10 und dem eingefüllten und ausgehärteten Knochenzement 40. Die äußere Hülle 35 der Vorrichtung 10 ist zur Außenhülle 5 des Gelenkspacers 1 geworden und enthält weiterhin die Entlüftungsöffnungen 37 und die Einfüllöffnungen 36.

Die Vorrichtung 10 bzw. der Gelenkspacer 1 kann zusätzlich zu den vorhandenen Öffnungen 36, 37 Austrittsöffnungen 39 aufweisen, durch die ein Wirkstoff aus dem Inneren des Gelenkspacers 1 nach außen zum Patienten gelangen kann. Ein Wirkstoff, beispielsweise umfassend ein oder mehrere Antibiotika, kann im Rahmen einer septischen Wechseloperation dem Knochenzement 40 vor dem Einfüllen beigegeben werden, um gezielt die vorhandenen Keime zu bekämpfen.

Die Austrittsöffnungen 39 müssen sich nicht von den Entlüftungsöffnungen 37 unterscheiden, vielmehr können Entlüftungsöffnungen 37 auch als Austrittsöffnungen 39 dienen und umgekehrt. Es ist jedoch vorteilhaft, wenn an mehreren Stellen, ggf. über die gesamte Oberfläche verteilt, Austrittsöffnungen 39 vorhanden sind, insbesondere auch an Stellen, an denen Entlüftungsöffnungen 37 nur wenig effektiv wären wie z. B. nahe der Einfüllöffnungen 36.

### Bezugszeichenliste

| | |
|---|---|
| Gelenkspacer | 1 |
| Kopfbereich | 2 |
| Schaftbereich | 3 |
| Halsbereich | 4 |
| Außenhülle | 5 |
| Vorrichtung | 10 |
| Kopf | 11 |
| Hals | 12 |
| Schaft | 13 |
| Fortsatz | 14 |
| Fixierungseinrichtung | 15 |
| Stift | 17 |
| Weiterer Stift | 17' |
| Formschlusselemente | 18 |
| Weitere Formschlusselemente | 18' |
| Nut | 19 |
| Umschalteinrichtung | 20 |
| Öffnungsposition | 21 |
| Schließposition | 22 |
| Gehäuseteil | 23 |
| Längsachse | 25 |
| Rotierende Einheit | 26 |
| Sperrelement | 27 |
| Weiteres Sperrelement | 27' |
| Skala | 28 |
| Knopf | 30 |
| Erste Aussparung | 31 |
| Zweite Aussparung | 32 |
| Federelement | 33 |
| Hohlraum | 34 |
| Äußere Hülle | 35 |
| Einfüllöffnung | 36 |
| Entlüftungsöffnung | 37 |
| Applikator | 38 |
| Austrittsöffnung | 39 |
| Knochenzement | 40 |

## Patentansprüche

1. Vorrichtung (10) zur Ermittlung eines benötigten Abstands eines Kopfbereichs (2) von einem Schaftbereich (3) eines Gelenkspacers (1), umfassend einen Kopf (11) und einen Schaft (13), die in unterschiedlichen Abständen zueinander positionierbar sind, wobei die Vorrichtung (10) ferner eine Fixierungseinrichtung (15) zum Fixieren eines Abstands zwischen dem Kopf (11) und dem Schaft (13) aufweist.

2. Vorrichtung (10) nach dem vorhergehenden Anspruch, **dadurch gekennzeichnet, dass** die Vorrichtung (10) ferner einen Hals (12) aufweist, der den Kopf (11) mit dem Schaft (13) verbindet.

3. Vorrichtung (10) nach einem der vorhergehenden Ansprüche, **dadurch gekennzeichnet, dass** der Kopf (11) einerseits und der Schaft (13) und/oder der Hals (12) andererseits getrennt voneinander vorliegen oder voneinander getrennt werden können.

4. Vorrichtung (10) nach einem der zwei vorhergehenden Ansprüche, **dadurch gekennzeichnet, dass** der Kopf (11) auf dem Hals (12) verschieblich ist, wobei die Fixierungseinrichtung (15) mindestens einen Stift (17) sowie eine Vielzahl an Formschlusselementen (18) aufweist, wobei der Stift (17) so mit einem oder zwei Formschlusselementen (18) in Kontakt gebracht werden kann, dass ein Verschieben des Kopfes (11) auf dem Hals (12) gesperrt ist.

5. Vorrichtung (10) nach dem vorhergehenden Anspruch, **dadurch gekennzeichnet, dass** angrenzend an die Vielzahl an Formschlusselementen (18) eine axial ausgerichtete Nut (19) angeordnet ist, wobei der Stift (17) in der Nut (19) in axialer Richtung verschoben werden kann, um den Abstand einzustellen.

6. Vorrichtung (10) nach einem der vorhergehenden Ansprüche, **dadurch gekennzeichnet, dass** die Fixierungseinrichtung (15) eine Umschalteinrichtung (20) zum Umschalten von einer Öffnungsposition (21) in eine Schließposition (22) umfasst, wobei in der Öffnungsposition (21) der Abstand zwischen Kopf (11) und Schaft (13) verändert werden kann und in der Schließposition (22) der Abstand zwischen Kopf (11) und Schaft (13) fixiert ist.

7. Vorrichtung (10) nach dem vorhergehenden Anspruch und Anspruch 2, **dadurch gekennzeichnet, dass** das Umschalten durch Rotation einer rotierenden Einheit (26) in Bezug zu einem Gehäuseteil (23) um eine Längsachse (25) des Halses (12) erfolgt.

8. Vorrichtung (10) nach einem der zwei vorhergehenden Ansprüche, **dadurch gekennzeichnet, dass** ein Sperrelement (27) vorhanden ist, welches beim Umschalten in die Schließposition (22) aktiviert wird und jede Bewegung zwischen Kopf (11) und Schaft (13) blockiert.

9. Vorrichtung (10) nach dem vorhergehenden Anspruch, **dadurch gekennzeichnet, dass** das Sperrelement (27) mit einem federnd gelagerten Knopf (30) verbunden ist, welcher mit zwei Aussparungen (31, 32) zusammenwirkt, wobei der Knopf (30) in einer ersten Aussparung (31) positioniert ist, wenn sich die Fixierungseinrichtung (15) in der Schließposition (22) befindet, wobei der Knopf (30) in einer zweiten Aussparung (32) positioniert ist, wenn sich die Fixierungseinrichtung (15) in der Öffnungsposition (21) befindet.

10. Vorrichtung (10) nach einem der sechs vorhergehenden Ansprüche, **dadurch gekennzeichnet, dass** die Fixierungseinrichtung (15) ferner mindestens einen weiteren Stift (17') aufweist, der sich ausgehend von dem Stift (17) in einer um 180° um die Längsachse (25) gedrehten Position befindet, und dass die Fixierungseinrichtung (15) ferner eine weitere Vielzahl an Formschlusselementen (18') aufweist, die sich ausgehend von der Vielzahl an Formschlusselementen (18) in einer um 180° um die Längsachse (25) gedrehten Position befindet.

11. Vorrichtung (10) nach einem der vorhergehenden Ansprüche, **dadurch gekennzeichnet, dass** die Vorrichtung (10) im Bereich des Kopfes (11) und/oder des Schafts (13) eine äußere Hülle (35) aufweist, die einen Hohlraum (34) nach außen hin begrenzt, wobei in der äußeren Hülle (35) eine Einfüllöffnung (36) zum Einfüllen von Knochenzement in den Hohlraum (34) angeordnet ist.

12. System, umfassend eine Vorrichtung (10) nach einem der vorhergehenden Ansprüche sowie
- mindestens einen zusätzlichen Kopf (11), der an dem Schaft (13) der Vorrichtung (10) positionierbar ist, wobei der Kopf (11) eine andere Größe aufweist als der Kopf (11) der Vorrichtung (10), und/oder
- mindestens einen zusätzlichen Schaft (13), an dem der Kopf (11) der Vorrichtung (10) positionierbar ist, wobei der Schaft (13) eine andere Größe aufweist als der Schaft (13) der Vorrichtung (10).

13. Verfahren zur Herstellung eines Gelenkspacers (1), bei dem ein Kopf (11) und ein Schaft (13) einer Vorrichtung (10) in einem gewünschten Abstand zueinander positioniert werden, der Abstand fixiert wird und Knochenzement (40) in den Kopf (11) und/oder den Schaft (13) gefüllt wird.

14. Verfahren nach dem vorhergehenden Anspruch, wobei eine äußere Hülle (35) der Vorrichtung (10) eine Außenhülle (5) des Gelenkspacers (1) bildet.

15. Verwendung einer verlorenen Schalung zur Herstellung des Gelenkspacers (1).
